# EUROPEAN PATENT SPECIFICATION

(11) **EP 3 669 190 B1**
(45) Date of publication and mention of the grant of the patent: **01.11.2023**
(21) Application number: 18765185.6
(22) Date of filing: 07.08.2018
(51) Int. Cl.: G01N 33/50, C12N 5/071

(54) **A RELIABLE AND REPRODUCIBLE INDUSTRIALISATION PROCESS FOR THE ELIMINATION OF AIR BUBBLES IN THE PRODUCTION OF AN ENGINEERED VASCULAR TISSUE**
ZUVERLÄSSIGES UND REPRODUZIERBARES INDUSTRIALISIERUNGSVERFAHREN ZUR BESEITIGUNG VON LUFTBLASEN BEI DER HERSTELLUNG EINES KÜNSTLICHEN GEFÄSSGEWEBES
PROCÉDÉ D'INDUSTRIALISATION FIABLE ET REPRODUCTIBLE POUR L'ÉLIMINATION DE BULLES D'AIR DANS LA PRODUCTION D'UN TISSU VASCULAIRE RECONSTRUIT

(30) Priority: 16.08.2017 IT 201700093925
(43) Date of publication of application: 24.06.2020
(73) Proprietor: 1Lab SA, 6928 Manno (CH)
(72) Inventor: TRESOLDI, Claudia, 6928 Manno (CH); VANTAGGIATO, Cristina, 6928 Manno (CH)
(74) Representative: Benedetto, Marco
(86) International application number: PCT/IB2018/055944
(87) International publication number: WO 2019/034966

(56) References cited:
- WO-A1-2015/134853
- WO-A2-03/082145
- PETER S. MCFETRIDGE ET AL: "Endothelial and Smooth Muscle Cell Seeding onto Processed Ex Vivo Arterial Scaffolds Using 3D Vascular Bioreactors", ASAIO JOURNAL, vol. 50, no. 6, 1 November 2004 (2004-11-01), pages 591-600, XP055181112, ISSN: 1058-2916, DOI: 10.1097/01.MAT.0000144365.22025.9B
- SEBASTIAN SCHUERLEIN ET AL: "A versatile modular bioreactor platform for Tissue Engineering", BIOTECHNOLOGY JOURNAL, vol. 12, no. 2, 1 February 2017 (2017-02-01), page 1600326, XP055521499, DE ISSN: 1860-6768, DOI: 10.1002/biot.201600326
- ALIDA ABRUZZO ET AL: "Using Polymeric Scaffolds for Vascular Tissue Engineering", INTERNATIONAL JOURNAL OF POLYMER SCIENCE, vol. 2014, 1 January 2014 (2014-01-01), pages 1-9, XP055470620, ISSN: 1687-9422, DOI: 10.1155/2014/689390
- ZHANG X ET AL: "Dynamic culture conditions to generate silk-based tissue-engineered vascular grafts", BIOMATERIALS, ELSEVIER, AMSTERDAM, NL, vol. 30, no. 19, 1 July 2009 (2009-07-01), pages 3213-3223, XP026130537, ISSN: 0142-9612, DOI: 10.1016/J.BIOMATERIALS.2009.02.002 [retrieved on 2009-02-20]

## Description

The present invention refers to a reliable and reproducible industrialisation method for the elimination of air bubbles in the production of an engineered vascular tissue for *in vitro* testing of medical products for human use and veterinary products for animal use.

It is known that the development of a medical product is a long and delicate process. In practice, in the development of a medical or veterinary product, whether a drug or medical device, before it can be used in human or animal subjects, it is important to determine its effects on the tissues it will come into contact with, to evaluate the biological safety aspects and the performance of the medical product and anticipate potential problems relative to its use.

In this context, the evaluation of the biological safety and performance of the medical product is wide-ranging and complex, and the evaluation of its interaction with the tissues of a single constituent material may not be considered in isolation from the overall design of the medical or veterinary product, which must be evaluated in its totality and in a manner which reproduces its conditions of use as faithfully as possible. Today, the evaluation of the biological safety and performance of a medical or veterinary product is based on testing *in vitro, ex vivo* and on animal model which have significantly differences with respect to the conditions of final use. In particular, the animal models have significant physiological differences which make difficult the transposition of the validity of the results to human subjects. Such differences are further appreciable when the objects of the evaluation are medical products for human use or veterinary product for animal use, the intended use of which is in the cardiovascular and peripheral vascular area, such as heart valves, stents, grafts, catheters, bandages or meshes.

Unfortunately, the evaluation of the interaction of medical products for human use or veterinary products for animal use with vascular tissue and blood to determine their biological safety and performance is critical, and the models currently in use have significant limitations and disadvantages, both anatomical and structural and in relation to the blood composition.

Furthermore, experiments on animals are the subject of intense controversy relating to whether the use of animals in biomedical experimentation is morally acceptable. This debate led to the establishment, as early as 1959, of the principle of the 3Rs, which is very relevant in the discussion and proposals of international research programmes. The principle of the 3Rs refers to three key concepts: replacement, reduction and refinement. The principle of the 3Rs states that biomedical research should, as far as possible: replace animal models with alternative models, reduce the number of animals used in any given experimental protocol, and refine or improve the experimental conditions to which the animals are subjected.

It is known that in the field of vascular tissue engineering, it is essential to be able to produce an endothelium which is both continuous (i.e. having a monolayer of confluent cells) and functional, i.e. composed primarily of endothelial cells (ECs).

The production of a continuous and functional endothelium is a critical factor in promoting scaffold endothelisation and assuring adequate performance of the engineered vascular constructs (composed of scaffold and cells) (tissue-engineered constructs), including prevention of thrombosis and stenosis once said constructs are implanted.

The generation *in vitro* of a vascular endothelium or engineered vascular construct/tissue comprises, in brief, the following the steps:
(1) seeding the endothelial cells into the lumen of the scaffold and adhesion of said cells,
(2) growth/proliferation and cell organisation in relation to the mechanical stimuli (such as the flow of a fluid) to which they are subjected, and
(3) generation of one or more layers of functional endothelial cells.

Given the crucial importance of the seeding step, many university research groups have invested in the recent years in the development of a variety of techniques for uniformly seeding the endothelial cells in the lumen of the scaffold and increasing the efficacy of said seeding. However, the results obtained so far are not completely satisfactory.

At the present time, two techniques are used to seed the endothelial cells: static and dynamic seeding.

The simpler static method consists in pipetting a suspension of cells directly onto the luminal surface of the scaffold and then incubating them for a short time in a Petri dish. This method is highly operator dependent, and is aggravated by the difficulty of obtaining a uniform layer of endothelial cells.

In contrast, the dynamic methods, mostly based on rotation, vacuum, electrostatic and magnetic fields, increase the efficacy of the cellular seeding, along with its uniformity and adhesion. Some of these dynamic methods may be transferred directly to and coupled with perfusion bioreactors, thus reducing the need to handle the scaffold. In this case, the scaffolds may be seeded as soon as they are seated inside the bioreactor chamber.

Other research groups, on the other hand, use a method of dynamic seeding employing continuous injection of a suspension of cells by means of a pump or syringe, or employing continuous perfusion of the lumen of the seeded scaffold with a culture medium using a peristaltic pump following injection of the suspension of endothelial cells. These methods require higher volumes of cell suspension than seeding methods employing pipetting or rotation, due to the need to fill the perfusion line volume in addition to the volume of the injection syringe, showing possible limitations in relation to the reduction of costs (cells and culture medium).

A completely different method from those discussed above is that of dripping the cell suspension into the lumen of a scaffold. In this case, the principle disadvantage consists in the low initial adhesion of the cells and low reproducibility of the method since it is highly operator dependent. A cell suspension dripping method is disclosed by McFetridge and coworkers (Bioreactors", ASAIO JOURNAL, vol. 50, no. 6, 1st of November 2004, pages 591-600). A method of producing ex vivo arterial scaffolds using decellularized scaffold of carotid arteries was used. Endothelial cells were seeded into the lumen of the scaffold.

Furthermore, the choice of a method for connecting a perfusion circuit (perfusion method), necessary for the perfusion of a scaffold, principally tubular, to the bioreactor-scaffold system, must be adapted to the experimental setup.

Given the range of methods of seeding and of connection of a perfusion circuit (perfusion method) discussed above, strong limitations and disadvantages remain present.

Hence, there is an evident need to provide a process comprising a method for seeding and connecting the perfusion circuit (perfusion method) to the bioreactor-scaffold system which is reproducible, reliable and efficacious, especially in the light of the application of said process for GLP (Good Laboratory Practice) certified tissue engineering laboratories, the scope of which is focused on advanced preclinical testing and clinical trials.

There is a need for the development of a process for the production of engineered vascular tissues/constructs having an endothelium which is both continuous (i.e. having a monolayer of confluent cells) and functional, wherein said process is simple, fast, efficacious, independent of the operator, well defined, highly reproducible and reliable, comprising: (1) a method for seeding cells, principally endothelial cells, inside the lumen of a scaffold, which assures the homogeneity and uniform adhesion of the endothelial cells, while eliminating air bubbles inside the scaffold; (2) a method for connecting the perfusion circuit to the bioreactor-scaffold system (perfusion method) which guarantees sterility and maintains the absence of air bubbles in the assembled system consisting of the perfusion circuit and the bioreactor-scaffold system.

There is also a need to develop *in vitro* engineered vascular constructs/tissues comprising a continuous and functional endothelium for conducting advanced preclinical testing and clinical trials, to avoid the use of laboratory animals for preclinical tests and clinical trials. Hence, a reliable, efficacious and reproducible procedure for the industrialisation of said *in vitro* engineered vascular constructs/tissues is necessary.

The Applicant, after a long and intense activity of research and development, has perfected a process comprising a seeding method and a method for connecting the perfusion circuit to the bioreactor-scaffold system, having the characteristics claimed in the appended claims.

Preferred embodiments of the present invention will be evident from the following detailed description.

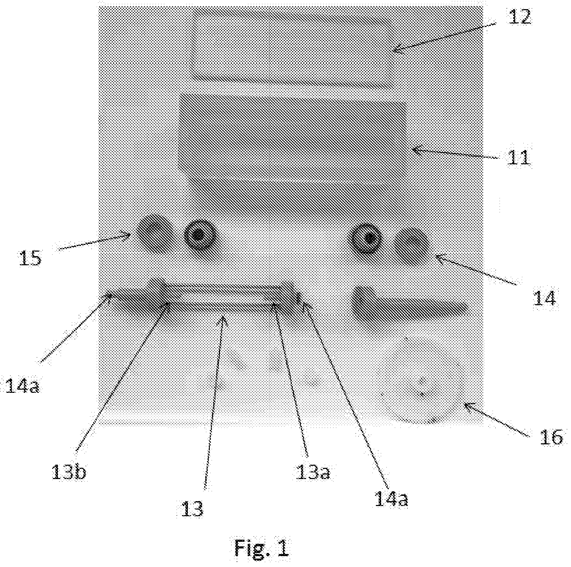

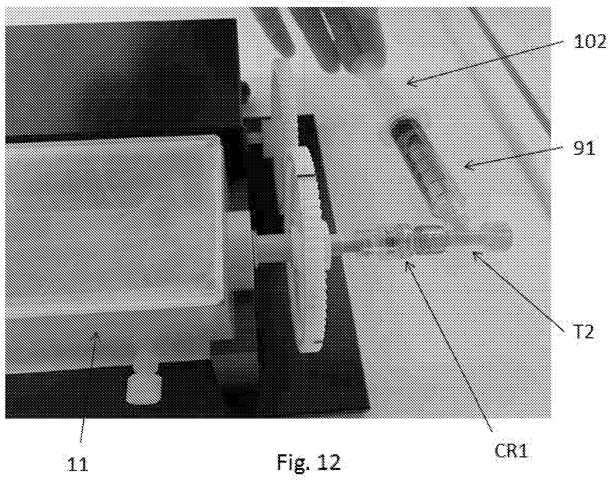

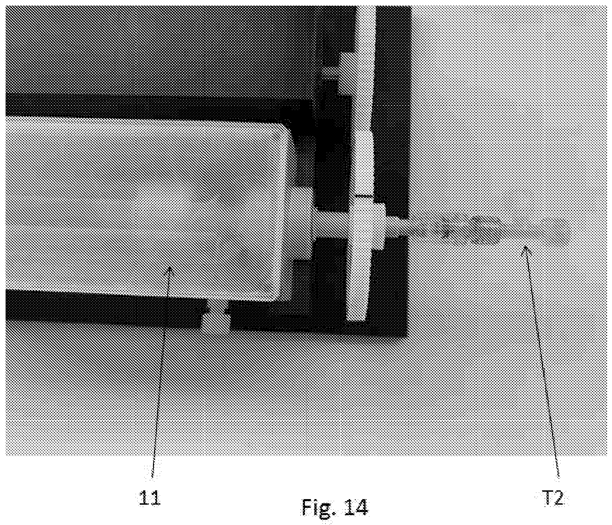

Figures 1-27 are described hereinafter in the present description.

In the context of the present invention by "continuous and functional endothelium" is meant an endothelium, with physiological-like behaviour, in which the endothelial cells are adjacent to each other, adhering to the scaffold and express the typical markers of endothelial cells, such as Von Willebrand factor (VWF), cluster of differentiation 31 (CD31), and vascular cell adhesion molecule 1(VCAM-1). In particular, by "continuous endothelium" is meant an endothelium having a monolayer of confluent cells.

In the context of the present invention, by "scaffold" is meant a biocompatible porous polymer substrate capable of promoting cell adhesion and growth, in the present case of endothelial cells. The polymer scaffold may be of synthetic or natural origin and formed of a single polymer or copolymers (set of polymers), such as electrospun silk fibroin or copolymers of PGA/PLA (polyglycolic acid/polylactic acid) or PGA/PCL (polyglycolic acid/polycaprolactone).

In the context of the present invention, by "endothelial cells" are meant the cells constituting the endothelium of a vascular tissue. Examples of endothelial cells are given by HAOECs (human aortic endothelial cells), HCAECs (human coronary artery endothelial cells), HMEVECs (human dermal microvascular endothelial cells) and HUVECs (human umbilical vein endothelial cells).

In the context of the present invention, by "engineered vascular construct" is meant a scaffold having a lumen coated principally by functional endothelial cells following a process of *in vitro* endothelisation. Said endothelial cells which cover the lumen of the scaffold constitute a continuous endothelium, i.e. an endothelium having a monolayer of confluent cells.

In the context of the present invention, by "culture medium" is meant the fluid of cell growth and maintenance specific to each type of cell. In particular, for the endothelial cells used in this specific case (HUVECs - Human Umbilical Vein Endothelial Cells, acquired from Sigma Aldrich, code 200-05n), the culture medium is Endothelial Growth Medium (EGM, Sigma Aldrich, 211-500). The EGM contains foetal bovine serum (2%), adenine (0.2 µg/ml), ammonium metavanadate (0.0006 µg/ml), amphotericin B (0.3 µg/ml), calcium chloride 2H₂0 (300 µg/ml), choline hydrochloride (20 µg/ml), copper sulphate 5H₂0 (0.002 µg/ml), trioptic acid DL-6,8 (0.003 µg/ml), folinic acid (calcium) (0.6 µg/ml), heparin (4 µg/ml), hydrocortisone (2 µg/ml), L-aspartic acid (15 µg/ml), L-cysteine (30 µg/ml), L-tyrosine (20 µg/ml), manganous sulphate monohydrate (0.0002 µg/ml), ammonium molybdate 4H₂0 (0.004 µg/ml), nicotinamide (8 µg/ml), nickel chloride 6H₂0 (0.0001 µg/ml), penicillin (60 µg/ml), phenol red sodium salt (15 µg/ml), potassium chloride (300 µg/ml), putrescine dihydrochloride (0.0002 µg/ml), pyridoxine hydrochloride (3 µg/ml), sodium metasilicate 9H₂O (3 µg/ml), sodium sulphate 7H₂O (200 µg/ml), sodium selenite (0.01 µg/ml), streptomycin sulphate (100 µg/ml), thiamine hydrochloride (4 µg/ml) and zinc sulphate 7H₂O (0.0003 µg/ml). The fresh culture medium is the sterile, never previously used medium as supplied directly by the manufacturer. By a "warm culture medium" is meant a culture medium previously heated to a temperature between 30°C and 45°C, preferably to 37°C.

The process considered in the present invention comprises a seeding method and a method for connecting a bioreactor to a perfusion circuit for scaffolds (perfusion method), preferably tubular scaffolds, for the engineering of vascular tissue with consequent production of vascular grafts (engineered vascular constructs/tissues) for testing medical products. Said process, comprising the seeding method and the method for connecting a perfusion circuit for bioreactor-scaffold systems (perfusion method), advantageously assures the accurate removal of air bubbles from the system described below and thus optimises the reproducibility of the process. Furthermore, by reducing the risk that air bubbles come into contact with the endothelial cells, it prevents damage to the endothelial cells and makes it possible to obtain a confluent monolayer of endothelial cells adhering to the lumen of the scaffold (continuous and functional endothelium). In the case in question, said method of seeding and connecting a perfusion circuit for scaffolds (perfusion method) applies to a preferably tubular scaffold of electrospun silk fibroin in a bioreactor for perfusion.

The process according to the present invention overcomes the limitations of currently available models and satisfies the 3R criteria inasmuch as it offers a valid alternative to the use of animal models.

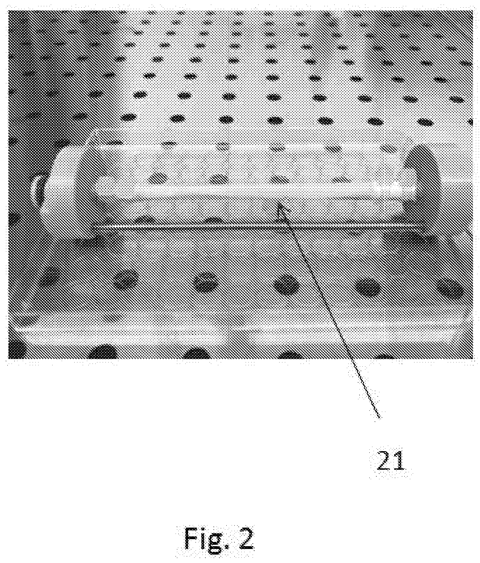

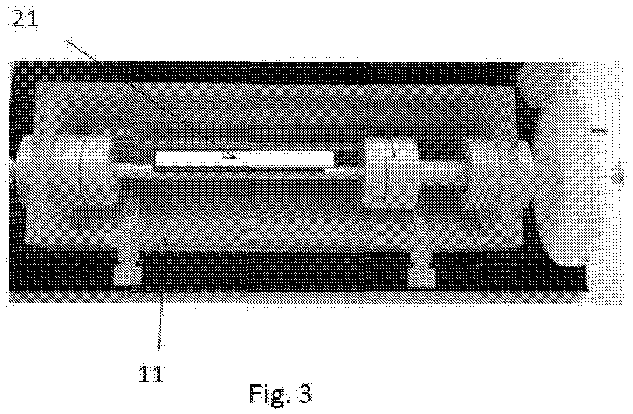

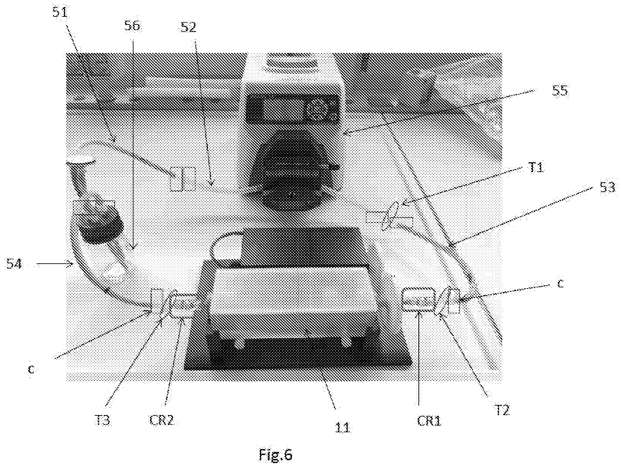

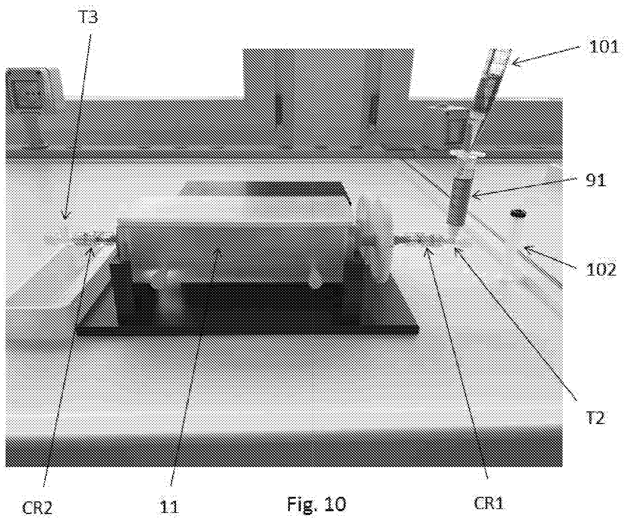

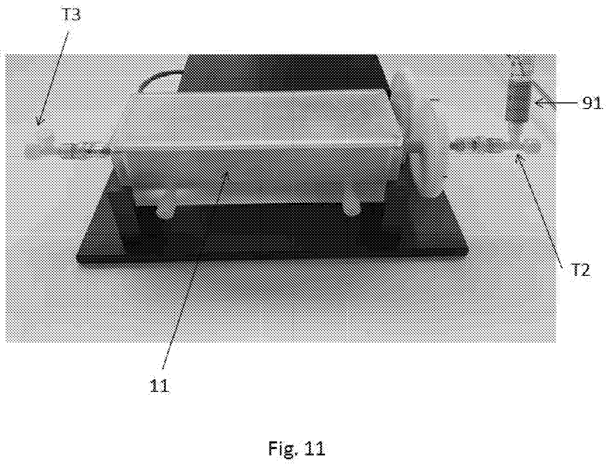

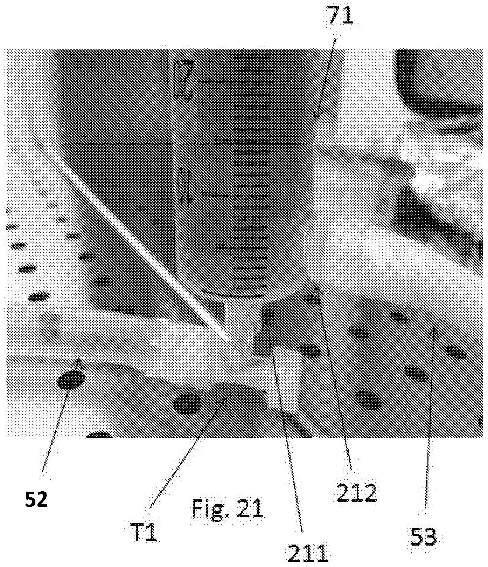

The present invention relates to a process for the production of an engineered vascular tissue or construct, preferably a scaffold (Fig. 2, 21) having a lumen coated with a functional and continuous endothelium having a monolayer of confluent cells, preferably usable for testing medical or veterinary models, wherein said process includes the application of:
- a method for seeding an endothelial cell culture into the lumen of a scaffold (21) to yield a seeded scaffold (21); said seeded scaffold (21) being present inside a bioreactor (11), to yield a seeded bioreactor (Fig. 3, 11)-scaffold (21) system;
wherein said seeding method comprises the step of:
- releasing said endothelial cell culture in the form of a cell suspension comprising a fresh culture medium and endothelial cells inside a container (Fig. 10, 91) mounted on a T connector (Fig. 10, T2) located upstream of the bioreactor (11) by means of a rotary connector (Fig. 10, CR1); followed by
- releasing said endothelial cell culture into the lumen of the scaffold (21) inside the bioreactor chamber (11) with a continuous flow such that the flow velocity permits said cell suspension to descend into the T connector (T2) without generating air bubbles and push the air bubbles inside the lumen of the scaffold (21) towards an opening of a T connector (Fig. 10, T3) located downstream of the bioreactor (11) enabling them to exit;
and successively,
- a perfusion method with a fresh culture medium having a temperature between 30°C and 45°C, preferably 37°C, of the endothelial cells present in the lumen of said seeded scaffold (21); said perfusion method being realised by the connection of a perfusion circuit (Fig. 6; 51, 52, 53, 54, 55, and 51-56 or 51-57 and BT) to said seeded bioreactor(11)-scaffold (21) system;
wherein said perfusion method comprises a step of:
- partly filling an element for the removal of the air bubbles (71 or BT) present in the perfusion circuit with said fresh culture medium, wherein said element for the removal of the bubbles (71 or BT) comprises a chamber, a cap closing said chamber, an inlet access (211) and an outlet access (212), wherein said element for the removal of the air bubbles (71 or BT) has a volume and wherein a first part of said volume if filled with said fresh culture medium and wherein a second part of said volume is filled with air, said second part of said volume having the function of trapping the air bubbles present in said fresh culture medium which flows through said inlet access (211) and said outlet access (212).

With the aim to illustrate preferred embodiments, the proposed technical solution represented by the process which the subject matter of the present invention is, for ease of comprehension, divided into the two methods described separately below in detail: (1) method for seeding a cell culture into the lumen of a scaffold, preferably a tubular scaffold; (2) method for connecting a perfusion circuit to a bioreactor-scaffold system (perfusion method).

### (1) Method for seeding a cell culture into the lumen of a scaffold according to a preferred embodiment.

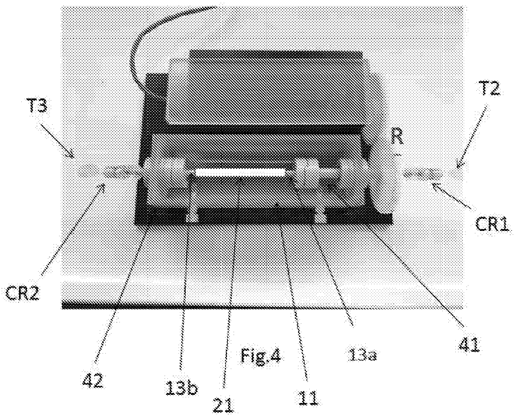

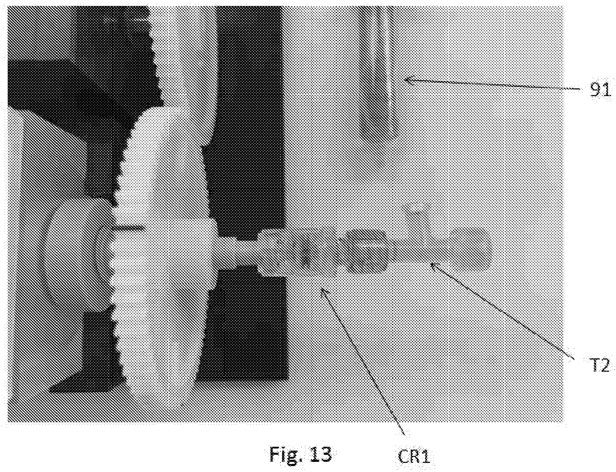

The method for uniformly seeding endothelial cells for a bioreactor-scaffold system comprises a plurality of steps preformed sequentially and in sterile conditions:
1.1 A scaffold (Fig. 2; 21), preferably a tubular scaffold, for example a tubular scaffold of an electrospun silk fibroin, mounted on the mountings of a scaffold support (Fig. 1; 13, 13a, 13b) is seated inside the chamber of the bioreactor, to yield a bioreactor-scaffold system. At both the extremities of the bioreactor (upstream and downstream) are applied rotary connectors (Fig. 4; CR1, CR2) and T connectors (Fig. 4; T2, T3).

In the context of the present invention, by "bioreactor-scaffold system" is meant the assembly of the bioreactor and the scaffold (Fig. 3; 11, 21), preferably a tubular scaffold, for example a tubular scaffold of an electrospun silk fibroin, seated and mounted by means of the mountings of the scaffold support (Fig. 1; 13, 13a, 13b) and then located inside the bioreactor. The scaffold, preferably tubular, is held by the mountings of the scaffold support (which is hollow to enable perfusion of the scaffold) with self-tightening straps, after having protected the scaffold, in this case made of electrospun silk fibroin, with sterilised teflon tape.

The scaffold support 13 is inserted into the bioreactor 11 in such a way that the inlet upstream of the chamber of the bioreactor coincides (Fig. 4; CR1, 41) with one end of the scaffold (Fig. 4; 13a) and the downstream opening of the bioreactor chamber (Fig. 4; CR2, 42) coincides with the other end of the scaffold (Fig. 4; 13b). This ensures that the scaffold 21 is perfectly coaxial with respect to the perfusion path generated by the hollow scaffold support. The longitudinal axis of the bioreactor is defined as the major axis along which the scaffold installed inside the bioreactor is oriented.

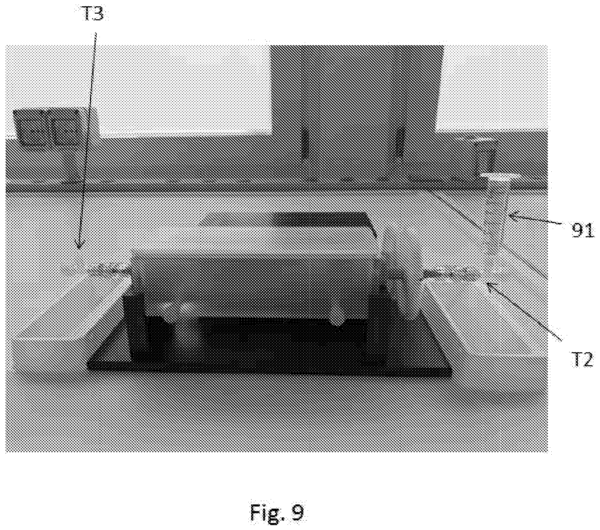

1.2 The scaffold is preconditioned with fresh culture medium injected into the lumen of the scaffold seated inside the bioreactor, using a syringe with luer-lock attachment engaged with one of the two ends of the bioreactor via T connector (Fig. 9; T2). The open ends of the connectors located upstream and downstream of the bioreactor are then closed with caps to prevent the lumen of the scaffold emptying. Furthermore, fresh culture medium is inserted into the chamber of the bioreactor until the scaffold seated inside it is completely covered. In this way the scaffold seated inside the chamber of the bioreactor is preconditioned, preferably for 1 hour at 25°C, with fresh culture medium both inside (in the lumen) and outside.

1.3 Following the preconditioning of the scaffold, inside the bioreactor preconditioned with culture medium injected into the lumen, it is emptied preferably with a sterile pipette, and the culture medium previously inserted into the chamber is removed preferably with a sterile pipette. The residual culture medium in the connectors (rotary and T) upstream and downstream of the bioreactor is removed by vacuum using a pipette, without collapsing the scaffold.

1.4 The T connectors located upstream (Fig. 9, T2) and downstream (Fig. 9; T3) of the ends of the bioreactor, containing the scaffold supported on the mountings, are turned so that the upper opening turned upwards (by 90° relative to the plane in which the bioreactor-scaffold system lies). The lateral openings of the T connectors located downstream (T3) and upstream (T2) of the bioreactor is then closed with a cap. A container, preferably a syringe, is then mounted on the opening of the T connector turned upwards (Fig. 9, T2) located upstream of the bioreactor, (Fig. 9; 91) with a luer-lock attachment of capacity, for example, of 5ml, with its plunger removed. Instead, the opening of the T connector (Fig. 9; T3) located downstream of the bioreactor is kept open (Figure 9).

1.5 With a pipette, preferably a sterile plastic pipette of capacity, for example, of 25ml (Fig. 10; 101), a cell suspension consisting of fresh culture medium and endothelial cells (e.g. HUVECs) is drawn from a container in which it has been prepared. Subsequently, the drawn cell suspension is released into the container or syringe (Fig 10; 91) mounted on the T connector (Fig. 10; T2) upstream of the bioreactor by means of the element (Fig. 10; CR1). The cell suspension must be released, with the pipette (Fig. 10; 101), for example of 25ml, with a continuous flow so that the flow speed enables the cell suspension to descend into the T connector (Fig. 10; T2) without generating air bubbles and drive any air bubbles inside the scaffold towards the opening of the T connector T3 located downstream (Fig. 10; T3) of the bioreactor 11 and thus exit the system (Figure 10).

1.6 When the cell suspension, loaded by means of the syringe, reaches the open end of the T connector T3 located downstream of the bioreactor without air bubbles, the opening of the T connector T2 located upstream of the bioreactor is closed with a cap (Figure 11).

1.7 The syringe (91) containing the residual cell suspension is then rotated by around 90° relative to the plane (Figure 12) in which it lies; in this position, the plunger of the syringe (102) is re-inserted into the open end of the syringe (Figure 12) so that only the black insulating extremity is inserted so as not to create pressure inside the scaffold. The syringe (91) may then be unscrewed from the T connector T2 located upstream of the bioreactor without forming air bubbles (Figure 13) and the end of the connector closed with a cap (Figure 14).

1.8 Warm fresh culture medium (as defined above) is added into the chamber of the bioreactor until the seeded scaffold inside the chamber of the bioreactor is halfway immersed in the culture medium.

1.9 The scaffold is then rotated continuously around its longitudinal axis, for instance with a speed of rotation between 1.5 and 2 rpm, for 24 hours. The rotation enables uniform adhesion of the cells to the lumen of the scaffold, keeps the scaffold itself continually wet with the culture medium inside the chamber of the bioreactor and allows nutrients to flow from the culture medium inside the chamber (outside the scaffold) to the cell suspension seeded inside the lumen of the scaffold.

1.10 Incubate, preferably for 24 hours at 37°C with 5% CO₂, the scaffold seated inside the chamber of the bioreactor (under rotation).

Advantageously, the seeding method developed by the Applicant maintains the sterility of the system. Furthermore, this seeding method which is the subject matter of the present invention is fast, reproducible, and advantageously enables preparation of a scaffold having the surface of the lumen (internal) with endothelial cells adhering uniformly and homogeneously along the entire length of the scaffold (from the proximal to the medial up to the distal part). The seeding method according to the present invention allows the cells to be seeded while both eliminating any air bubbles already present in the bioreactor-scaffold system and preventing the formation of new ones, thus preventing damage to the cells. In practice, each step of the present method is standardised and reproducible, and optimises the time and cost of the procedure.

### Experimental evidence of the seeding method

To demonstrate the efficacy of the seeding method according to the present invention, the following analyses were performed.

The vitality of the cells adhering to the scaffold was assayed with resazurin as reactant (tradename Alamar Blue, IUPAC name 7-hydroxy-10-oxido phenoxazine-10-ium-3-one, CAS 550-82-3). This assay consists in a metabolic reaction which makes it possible to quantify the vitality of the cells due to the redox reaction of the indicator (resazurin) which reduces to resofluorine, a fluorescent compound which turns pink in the presence of the reductive environment of a living cell. After 24 hours of adhesion, the seeded scaffold is removed from the mountings. The scaffold is then sectioned (cut) in three zones of 2cm length each, in relation to the site of injection of the cell suspension: proximal, medial and distal. Each section is then divided into 4 parts of approximately 1cm² each. For the resazurin assay, 3 samples are selected, each representative of each region (proximal, medial and distal) of the scaffold with adhering endothelial cells. Each sample is placed in a well of a 24 well plate and incubated with 1ml of a 0.02 mg/ml solution of resazurin sodium salt (Sigma Aldrich, R7017) with fresh culture medium preferably for 3 hours at 37°C with 5% CO₂. The reaction between the 0.02 mg/ml solution of resazurin sodium salt with fresh culture medium and the scaffold sample (with the adhering endothelial cells) is analysed by measuring the A.U. (arbitrary unit of fluorescence) at 590 nm with a spectrofluorometer.

A further analysis is the amount evaluation of the genomic DNA present in the cells adhering to the same scaffold samples previously used for the resazurin assay. The genomic DNA is extracted from the adhering cells of the scaffold by means of lysis and quantified using the Quant-iT^{™} PicoGreen^{™} dsDNA Assay (P7589, Invitrogen, Molecular Probes) in which a fluorescent colourant of the nucleic acids (PicoGreen) allows by a standard reference curve to determine the concentration of genomic DNA in the solution.

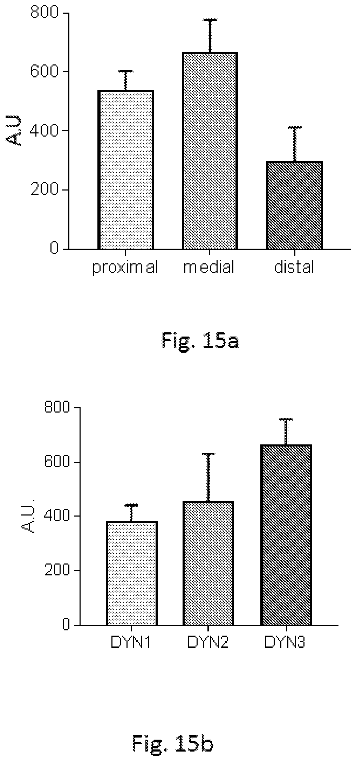

Figures 15A and 15B show graphically the values obtained from the resazurin assay for the samples representative of each region (proximal, medial and distal) of a scaffold seeded with endothelial cells and incubated for 24 hours in three separate experiments (denominated DYN1, DYN2 e DYN3). The graphs in Figures 15A and 15B show good adhesion and vitality of the endothelial cells.

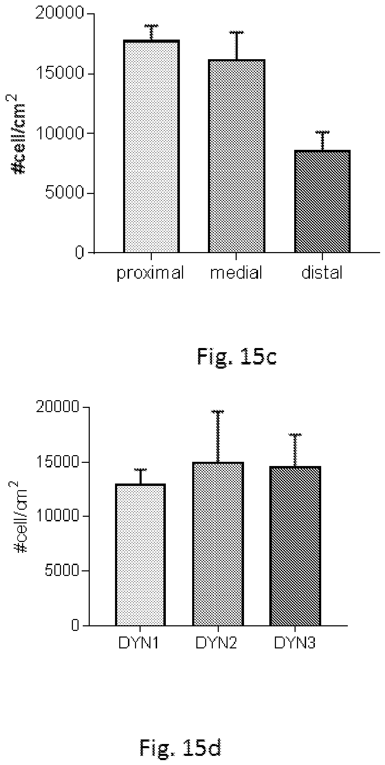

These data are confirmed by the quantification of the genomic DNA (Figures 15C and 15D) calculated in consideration of the fact that an endothelial cell contains around 7pg of genomic DNA.

No significant difference in cell vitality was found between the various proximal, medial and distal sections of the scaffold seeded with endothelial cells. In particular, the vitality and number of the cells are comparable along the length (principal axis of the scaffold) at its proximal, medial and distal portions.

This evidence was supported by means of costaining with DAPI (4',6-Diamidino-2-Phenylindole, Dihydrochloride, D1306, ThermoFisher scientific) and Rhodamine-Phalloidin (R415, ThermoFisher scientific) samples representative of each region (proximal, medial, distal) of a scaffold seeded with endothelial cells and incubated for 24 hours. The two reagents, DAPI and Rhodamine-Phalloidin, are specific for detection of the nuclei and for actin filaments (F-actin) respectively, both morphological components of a living cell, which can be seen after staining, using a fluorescence microscope or confocal microscope. These results after 24 hours of growth demonstrate that the endothelial cells are vital and distributed uniformly over the lumen of the scaffold. In particular, the results demonstrate a cell confluence of 90%.

Furthermore, on samples representative of each region (proximal, medial, distal) of a scaffold seeded with endothelial cells and incubated for 24 hours, gene expression analyses were run for the typical markers of the endothelial cells: for example Von Willebrand factor (VWF), cluster of differentiation 31 (CD31) and vascular cell adhesion molecule 1(VCAM-1). The gene expression was evaluated by extracting the total RNA from the cells (in this case endothelial cells) and quantifying it after retrotranscription to cDNA with specific Taqman Gene Expression Assay (ThermoFisher Scientific) using the real-time PCR technique. Functional levels of gene expression of the above markers are an indication of the good functionality and vitality of the cells adhering to the lumen of the scaffold.

Finally, on samples of a scaffold seeded with endothelial cells according to the present invention, H&E staining "Haematoxylin and Eeosin staining" was run to evaluate the distribution of the cells and their morphology, and an immunofluorescence assay was run for the specific endothelial functionality markers.

In conclusion, the seeding method according to the present invention has been shown to be efficacious inasmuch as it guarantees homogeneous, uniform and reproducible seeding of vital endothelial cells over the entire length of the lumen.

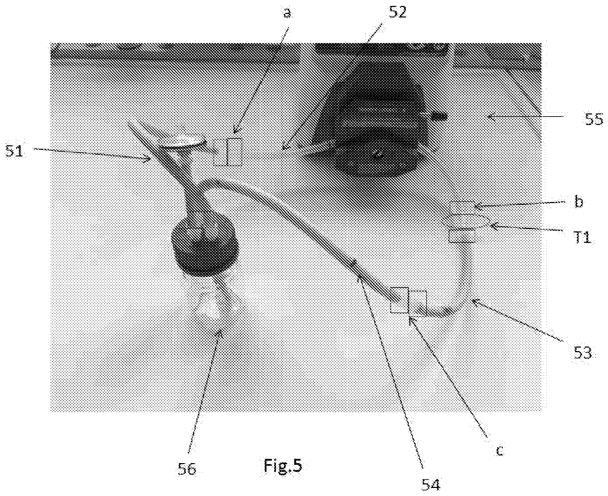

(2) Method for connecting a perfusion circuit to a bioreactor-scaffold system (perfusion method) according to a first embodiment (Figures 5-8, 18-21).

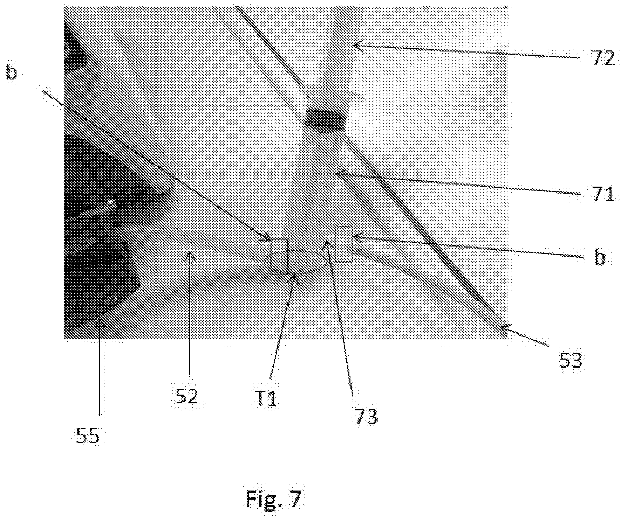

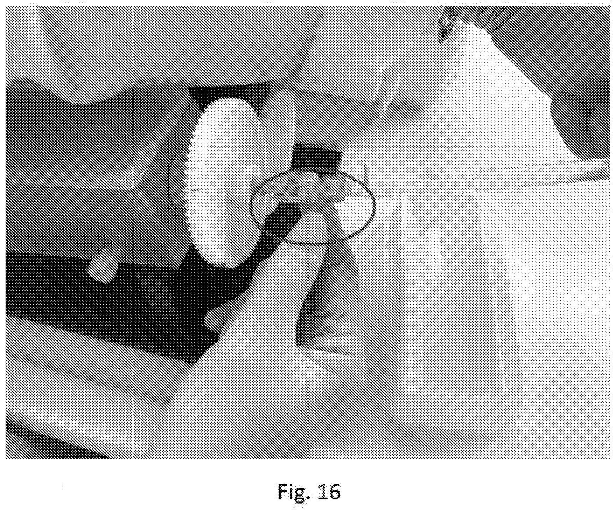

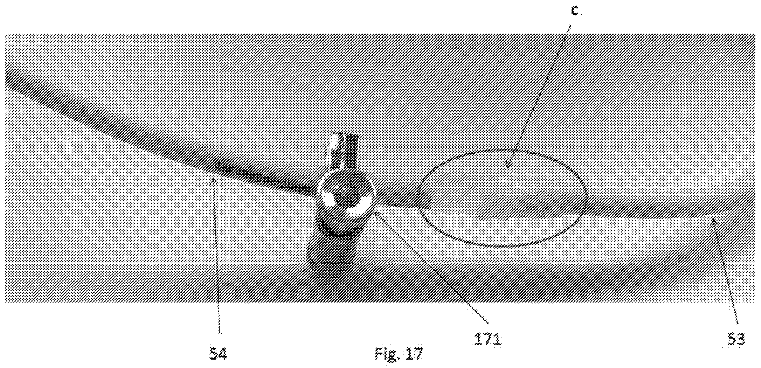

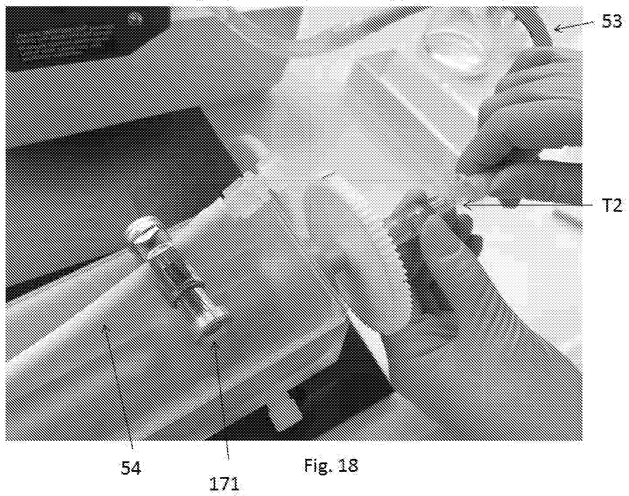

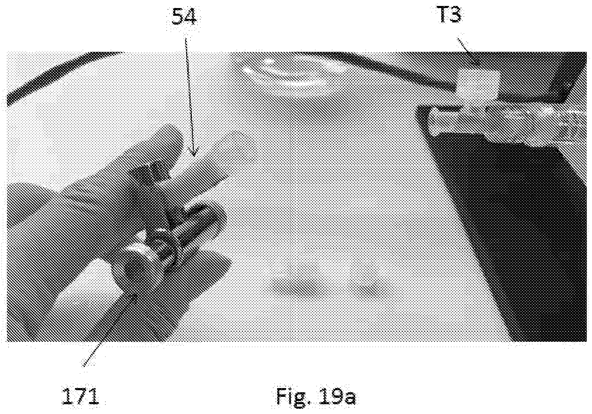

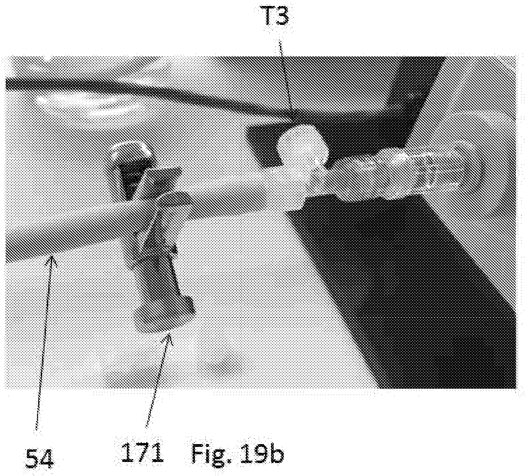

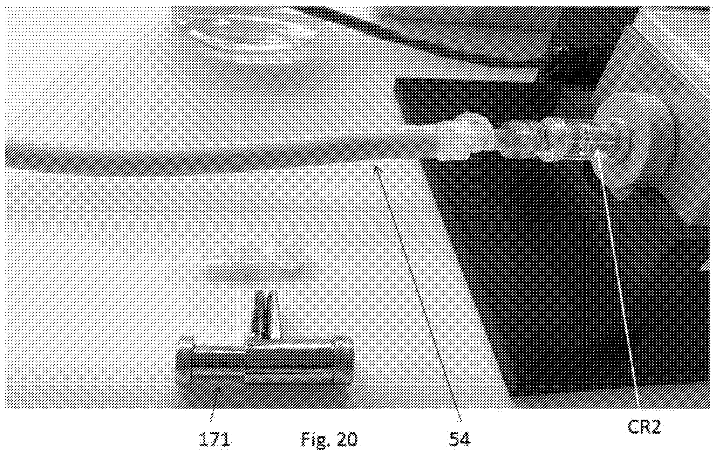

The connection method is based on the following sequence of steps, following seeding (method for seeding a cell culture in the lumen of a scaffold according to the embodiment described above at point (1)) after 24 hours of adhesion of the endothelial cells:
2.1 Locate the tube or pump loop tubing (Fig. 5; 52) of the closed perfusion circuit under the head of the peristaltic pump (Fig. 5; 55), which once activated generates a peristaltic force capable of aspiring fluids, in this case warm fresh culture medium. The closed perfusion circuit is filled by means of aspiration, by the tube (Fig. 5; 51) of the perfusion circuit connected to the reservoir (Fig. 5; 56), of the warm fresh culture medium previously poured into the reservoir which is then closed. Fill all the tubes of the perfusion circuit with the warm fresh culture medium until the fresh culture medium returns to the reservoir via tube 54 (Fig. 5; 54) of the closed perfusion circuit. Position the bioreactor-scaffold system in the same conditions of sterility as the perfusion circuit.
2.2 Open the upper and lateral ends of the T connector T2 located upstream of the bioreactor.
2.3 When the caps are removed from the upper and lateral ends of the T connector T2 located upstream of the bioreactor, the formation of bubbles, if any, is compensated by the manual addition (preferably with a pasteur) of an equal volume of warm fresh culture medium (Fig. 16).
2.4 Occlude tube 54 of the closed perfusion circuit, preferably with a clamp (Fig. 17; 171) located proximally to the connector between tube 54 and tube 53 of the perfusion circuit itself (Fig. 17). Take care to keep the head of the pump closed to prevent tube 53 of the closed perfusion circuit emptying.
2.5 Keep the tube 53 of the closed perfusion circuit in vertical position, unscrew the connector between the tube 53 and 54 of the perfusion circuit and cap preferably the tube 54 of the perfusion circuit with a cap.
2.6 Screw the tube 53 of the perfusion circuit to the open lateral end of the T connector T2 upstream of the bioreactor next to its lateral access. The connector upstream of the bioreactor must be kept in vertical position at all times (Fig. 18).
2.7 Open the T connector T3 downstream of the bioreactor by unscrewing the cap of the lateral opening.
2.8 Remove the cap from the connector of the tube 54 of the perfusion circuit (Fig. 19A) and screw it onto the lateral opening of the T connector downstream of the bioreactor (Fig. 19B).
2.9 Remove the clamp 171 occluding the tube 54 of the perfusion circuit (Fig. 20).
2.10 Fill the air bubble removal element (Fig. 21, 71), in the present invention defined with the technical term bubble trap. The bubble trap consists of an element represented by a closed chamber with a cap and having two accesses with the function of inlet and outlet. The chamber of the bubble trap contains a volume of fluid (in this case warm fresh culture medium) and a volume of air which traps any bubbles in the fluid in perfusion which flows through the two accesses of the chamber of the bubble trap.
   Fill the bubble trap with warm fresh culture medium so as to leave a certain volume of air and close the chamber and its two accesses with their caps.
2.11 Connect the bubble trap to the perfusion circuit previously connected to the bioreactor-scaffold system as follows (Fig. 7):
   a. close the tube 53 of the perfusion circuit with a clamp located proximally to the connector which connect the tube 53 to the lateral end of the T connector T1 (located between tube 53 and tube 52 of the perfusion circuit) and unscrew it (Fig. 7; 52).
   b. preferably, close the tube 53 of the perfusion circuit with a cap. This operation prevents the tube 53 of the perfusion circuit from emptying.
   c. cap the lateral end of the T connector T1 (located between the tube 53 and the tube 52 of the perfusion circuit) and open its upper end.
   d. open the inlet access of the chamber of the bubble trap and connect it to the access of the vertical upper end of the T connector T1.
   e. open the outlet access of the bubble trap and connect it to the tube 53 of the perfusion circuit, taking care not to twist the tube.
   f. hold the bubble trap in vertical position,
   g. remove the clamp 71 from the tube 53 of the perfusion circuit as soon as it is connected to the chamber of the bubble trap. Turn on the pump and open the cap on the upper end of the T connector T2 upstream of the bioreactor to eliminate any air bubbles which may have formed in the tube 53 during the process preventing them from reaching the scaffold. The peristaltic for applied by the pump to the assembled system consisting of the perfusion circuit and the bioreactor-scaffold system enables perfusion of the scaffold.
   h. after this check, close the T connector T2 upstream of the bioreactor with its cap.

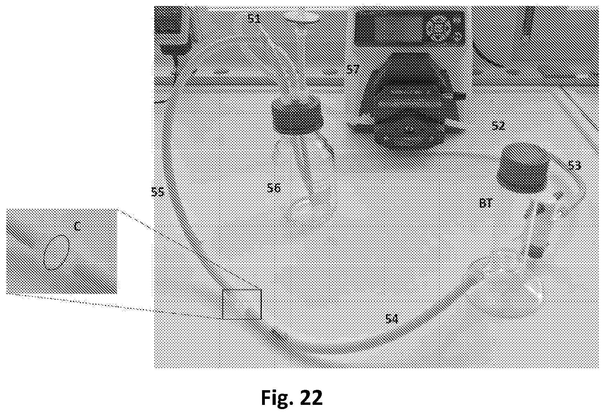

(3) Method for connecting a perfusion circuit to a bioreactor-scaffold system (perfusion method) according to a second preferred embodiment (Figures 22-27).

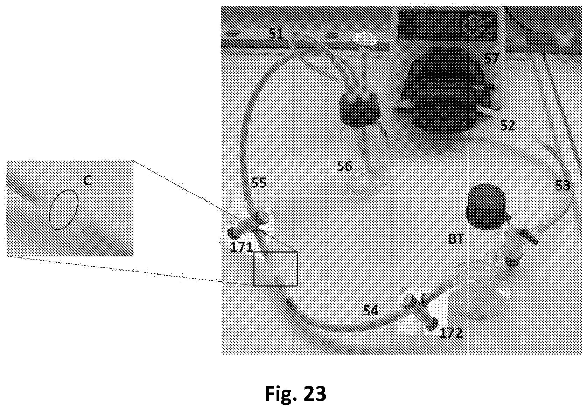

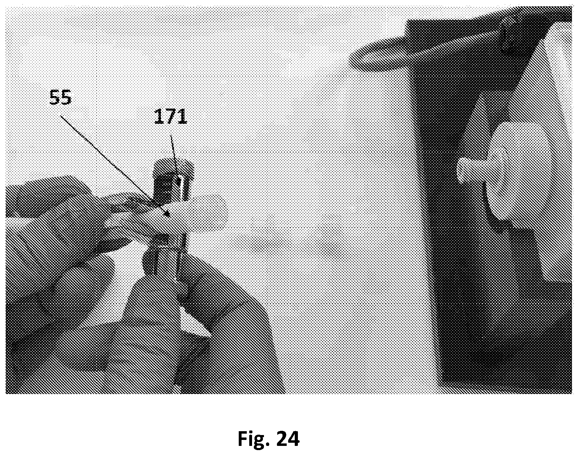

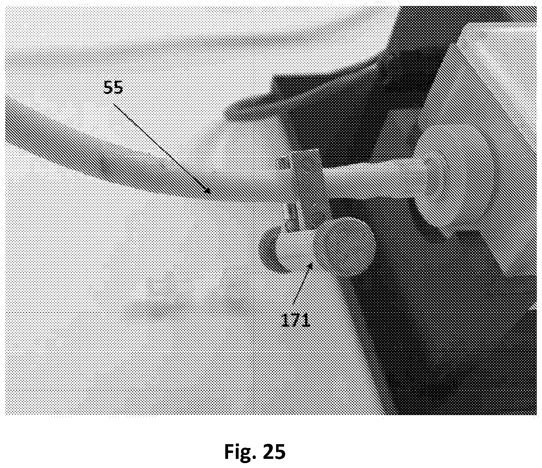

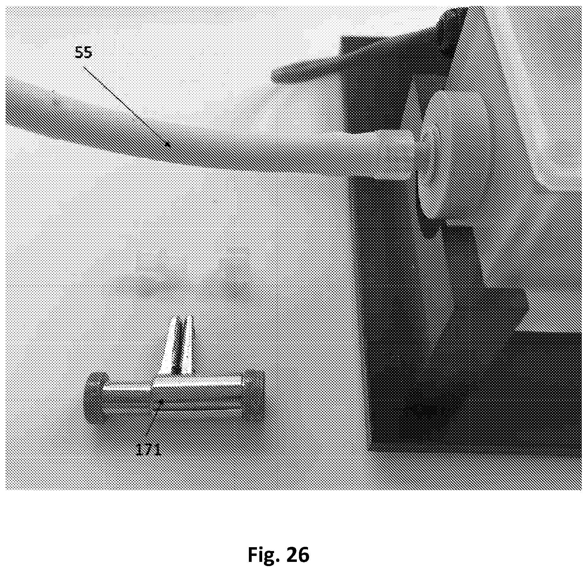

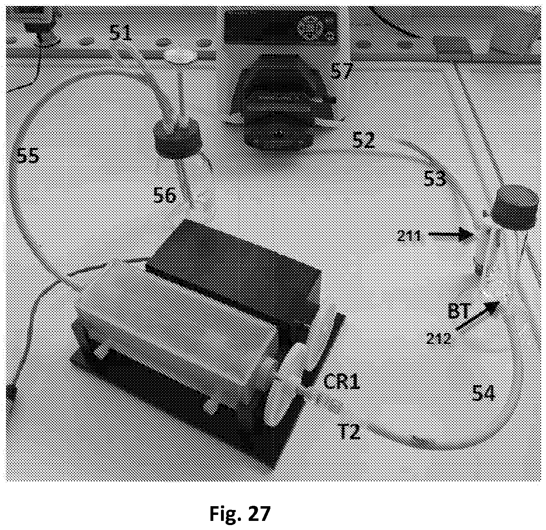

The connection method is based on the following sequence of steps, following seeding (method for seeding a cell culture in the lumen of a scaffold according to the embodiment described above at point (1)) after 24 hours of adhesion of the endothelial cells:
3.1 Connect the tubes of the perfusion circuit (Fig. 22; 51;
52; 53; 54; 55), the air bubble removal element (Fig. 22, BT), in the present invention defined with the technical term bubble trap, and the reservoir (Fig. 22; 56), while maintaining sterility. The air bubble removal element or bubble trap (BT) consists of an element represented by a closed chamber, preferably in glass, with a cap and having two asymmetrical accesses: the access with stopcock and tube clamp has the function of inlet (Fig. 27, 211) while the access with tube clamp only has the function of outlet (Fig. 27, 221). The chamber of the bubble trap contains a volume of fluid (in this case warm fresh culture medium) and a volume of air which traps any bubbles in the fluid in perfusion which flows through the two accesses of the chamber of the bubble trap.
3.2 Locate the pump loop tubing (Fig. 22; 52) of the closed perfusion circuit under the head of the peristaltic pump (Fig. 22; 57), which once activated generates a peristaltic force capable of aspiring fluids, in this case warm fresh culture medium. The closed perfusion circuit is filled by means of aspiration, by the tube (Fig. 22; 51) of the perfusion circuit connected to the reservoir (Fig. 22; 56), of the warm fresh culture medium previously poured into the reservoir (Fig. 22; 56) which is then closed. Fill all the tubes of the perfusion circuit, the bubble trap (Fig. 22, BT) and the reservoir (Fig. 22, 56) with a perfusion fluid, such as a warm fresh culture medium (as defined above) until the warm fresh culture medium returns to the reservoir via tube 55 (Fig. 22; 55) of the closed perfusion circuit. The BT and the reservoir must be filled as to leave a certain volume of air. In particular, the bubble trap (Fig. 22, BT) must be filled so that said chamber of the bubble trap has a first part of its volume filled with said fresh culture medium and a second part of its volume filled with air, said second part of said volume having the function of trapping the air bubbles present in the perfusion fluid (warm fresh culture medium) which flows through said inlet access (211) and said outlet access (212) of the bubble trap (BT)(Fig. 22).
3.3 Occlude the tube 54 (Fig. 23), preferably with a clamp (Fig. 23; 172) in a position proximal to the BT and close the stopcock of the BT (in a position perpendicular to the tubes of the perfusion circuit).
3.4 Position the bioreactor-scaffold system in the same conditions of sterility as the perfusion circuit.
3.5 Occlude the tube 55 (Fig. 23), preferably with a clamp (Fig. 23; 171; Fig. 17; 171) in a position proximal to the connection C with the tube 54 (Fig. 23; C). Open the upper and lateral ends of the T connector T2 located upstream of the bioreactor (Fig. 4; T2).
3.6 When the caps are removed from the upper and lateral ends of the T connector T2 (Fig. 4) located upstream of the bioreactor, the formation of bubbles, if any, is compensated by the manual addition (preferably with a pasteur) of an equal volume of warm fresh culture medium (Fig. 16).
3.7 Hold the tube 54 (Fig. 23) of the closed perfusion circuit in vertical position, unscrew the connector between the tube 54 (Fig. 23) and the tube 55 (Fig. 23) of the perfusion circuit and cap preferably the tube 55 of the perfusion circuit with a cap (Fig. 24).
3.8 Screw the tube 54 (Fig. 27) of the perfusion circuit to the open lateral end of the T connector T2 (Fig. 27) upstream of the bioreactor next to its lateral access. The connector upstream of the bioreactor must be kept in vertical position at all times (Fig. 18 or 27).
3.9 Open the T connector T3 (Fig. 4) downstream of the bioreactor by unscrewing the cap of the lateral opening.
3.10 Unscrew the rotary connector T3 together with the T connector CR2 (Fig. 4), holding the toothed wheel R (Fig. 4) stationary, and screw the connector of the tube 55 (Fig. 25) onto the lateral opening of the scaffold support 14a (Fig. 1) (Fig. 25).
3.11 Remove the clamp 171 occluding the tube 55 of the perfusion circuit (Fig. 26).
3.12 Place the seeded bioreactor-scaffold system connected to the perfusion circuit at around 37°C with around 5% CO₂, place the pump loop tubing 52 (Fig. 27) in a free position, open the stopcock of the bubble trap BT (Fig. 27) positioning it parallel to the tubes of the perfusion circuit, remove the clamp 172 (Fig. 27) and turn on the pump (Fig. 27, 57). The peristaltic for applied by the pump to the assembled system consisting of the perfusion circuit and the bioreactor-scaffold system enables perfusion of the scaffold.

Advantageously, the connection method (perfusion method) described herein, both in the first embodiment and in the second embodiment described above, enable connection of a perfusion circuit of a scaffold, preferably tubular, to the seeded bioreactor-scaffold system. This procedure prevents the formation of air bubbles and prevents the bubbles, should they form, reaching the scaffold seeded with endothelial cells of the bioreactor-scaffold system. Furthermore, any air bubbles already in the perfusion circuit do not reach the scaffold thanks to the presence of the bubble trap (Fig. 21, 71; Fig. 27, BT) in the circuit itself. In this way, the method developed by the applicant assures a complete lack of air bubbles in the lumen of the scaffold. This system satisfies the requisites imposed by the configuration of the bioreactor. All the details described herein are necessary to render the method operator independent and hence to assure reproducibility of the results during the industrial generation *in vitro* of a construct with functional endothelium. Furthermore, this methodology makes it possible to work in conditions of sterility, since all actions are simple. Furthermore, the fast, traceable procedure reduces the risk that air bubbles come into contact with the cells, thus preventing damage to the endothelial cells adhering to the lumen of the scaffold, preferably tubular. Said perfusion method makes it possible to obtain engineered vascular tissues/constructs having a scaffold with a lumen coated with an endothelium which is both continuous (i.e. having a monolayer of confluent cells) and functional.

### Experimental evidence of the connection method

The experimental analyses relative to the evaluation of the method for connecting the perfusion circuit to the bioreactor-scaffold system are the same as those applied to the evaluation of the seeding method for a cell culture in a scaffold, preferably tubular.

In the context of the present invention, by "perfusion circuit" (Fig. 5 and Fig. 22) is meant a set of: tubes (Fig. 5; 51- 54 or Fig. 22; 51-55), a reservoir (Fig. 5 or Fig. 22; 56), a peristaltic pump (Fig. 5; 55 or Fig. 22; 57) and an element for removing air bubbles (Fig. 21, 71 or Fig. 22, BT). Said element for the removal of air bubbles may be present in the perfusion circuit prior to connection of the perfusion circuit to the seeded bioreactor-scaffold system (second embodiment of the perfusion method) or alternatively may be inserted into the perfusion circuit after connection of the perfusion circuit to the seeded bioreactor-scaffold system (first embodiment of the perfusion method). Said tubes are of biocompatible material and connected to each other in such a way as to permit perfusion of the scaffold (Fig. 21 and Fig. 27), preferably tubular, seated inside the bioreactor 11, by means of the peristaltic pump (Fig. 5; 55, Fig. 22; 57) (in this case, Masterflex^{®}, L/S Digital Dispensing Pump Drives 07551-20, Cole-Parmer) with the Easy-Load II 77200-62 head (Masterflex, Cole-Parmer). With reference to the second embodiment of the perfusion method described above and represented in figure 27, the perfusion circuit principally consists of five tubes with internal diameter of 3/16": first aspiration tube 51 exiting from the reservoir, second tube 52 pump loop tubing, third tube 53 connecting the circuit to the bubble trap BT, fourth tube 54 connecting the BT to the T connector T2 upstream of the bioreactor-scaffold system, fifth tube 55 return to the reservoir 56 connected to the T connector T3 downstream of the bioreactor-scaffold system.

With reference to the first embodiment of the perfusion method described above and represented in figure 5, the tube 51 is connected to the pump loop tubing 52, the pump loop tubing 52 to the BT, the BT to the tube 53, the tube 53 to the T connector T2 upstream of the bioreactor-scaffold system, the tube 54 to the T connector T3 downstream of the bioreactor-scaffold system and to the reservoir 56.

The reservoir (Fig. 5 or Fig. 22, 56) is the element containing the warm fresh culture medium (for example, Endothelial Growth Medium EGM, Sigma Aldrich) from which the tube 51 (Fig. 5 or Fig. 22) aspirates and to which the tube 54 (Fig. 5) or 55 (Fig. 22) returns, maintaining the entire circuit and the seeded bioreactor-scaffold system in a closed loop. The reservoir (Fig. 5 or Fig. 22, 56) is at atmospheric pressure, thanks to a 0.22 µm filter present on the cap of the reservoir, which guarantees sterility of the air.

The following preferred embodiments RPn, as given below, are also disclosed.

RP1. A process for the production of vascular tissue, preferably a scaffold (Fig. 2; 21), for testing medical products, said process comprising:
- a method for seeding an endothelial cell culture in the lumen of a scaffold (Fig. 2; 21) to yield a seeded scaffold; said seeded scaffold being present inside a bioreactor (Fig. 4; 11), to yield a seeded bioreactor-scaffold system and, subsequently,
- a method for perfusing the endothelial cells present in the lumen of said seeded scaffold; said perfusion method consisting in the connection of a perfusion circuit (Fig. 6; 51, 52, 53, 54, 55 and 56) to said seeded bioreactor-scaffold system.

RP2. The process according to claim RP1, wherein said seeding method of an endothelial cell culture in the lumen of a scaffold comprises:
- installing a scaffold (21), preferably a tubular scaffold of electrospun silk fibroin, on the mountings of a scaffold support (Fig. 1; 13, 13a, 13b) and seated said scaffold support (13) with the scaffold (21) inside the chamber of the bioreactor (11), to yield a bioreactor-scaffold system (Fig. 4; 11, 21);
- injecting a fresh culture medium into the lumen of said scaffold (21) mounted on said scaffold support (13) inside the bioreactor chamber (11);
- adding said fresh culture medium inside the bioreactor chamber (11) in which is present said scaffold support (13) with the scaffold (21) previously injected with said culture medium;
- leaving, preferably for an interval of time between 1 hour and 18 hours at a temperature between 20°C and 30°C, preferably 25°C, said culture medium inside the lumen of the scaffold (21) and inside the bioreactor chamber (11) in which is present said scaffold support (13) with the scaffold (21) previously injected with said culture medium;
- clearing the interior of the lumen of the scaffold (21) and of the bioreactor chamber of the culture medium;
- releasing a cell suspension composed of said fresh culture medium and endothelial cells of the HUVECs type inside a container of the syringe type (Fig. 10; 91) mounted on the connector element (T2) located upstream of the bioreactor (11) by means of the element (CR1); said cell suspension is released into the lumen of the scaffold (21) present inside the chamber of the bioreactor (11) with a continuous flow so that the flow velocity permits said cell suspension to descend into the connector (T2) without generating air bubbles and push any air bubbles present inside the lumen of the scaffold (21) towards the opening of the connector (T3) located downstream of the bioreactor (11) to enable them to exit the system;
- adding said fresh culture medium inside the bioreactor chamber (11) in which is present said scaffold support (13) with the scaffold (21) containing said cell suspension inside it;
- incubating, preferably for 24 hours at 37°C in the presence of 5% CO₂, the scaffold (21) seated inside the bioreactor chamber (11).

RP3. The process according to RP1 or RP2, wherein said perfusion method of the endothelial cells in the lumen of said seeded scaffold comprises:
- providing a closed perfusion circuit (Fig. 5) comprising the tubes (Fig. 5; 51, 52, 53, 54, 55 and 56);
- occluding the tube (54) of the perfusion circuit with a closing element of the clamp type (Fig. 17; 171) in a position proximal to the connector (C);
- unscrewing the connector (C) located between the tube 53 and the tube 54 (Fig. 5; Fig. 17) of the perfusion circuit;
- screwing the tube (53) of the perfusion circuit to the open lateral end of the connector (T2) upstream of the bioreactor (Fig. 18) at its lateral access;
- opening the connector (T3) downstream of the bioreactor and unscrewing the cap of the lateral opening (Fig. 19a);
- connecting the tube (54) of the perfusion circuit to the lateral opening of the connector (T3) downstream of the bioreactor (Fig. 19b) and removing the closing element of the clamp type (Fig. 20; 171), while maintaining sterility.

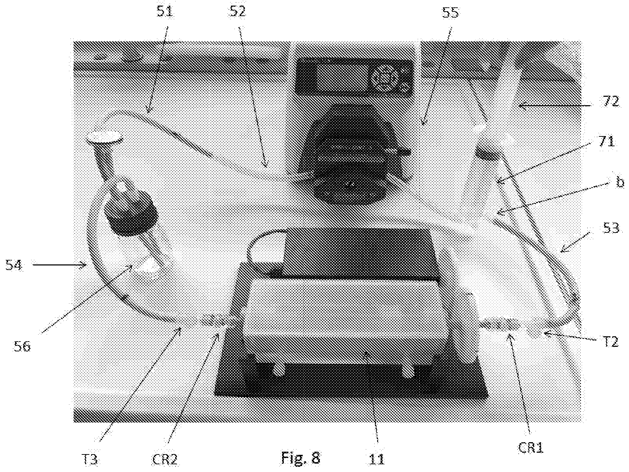

RP4. The process according to RP3, wherein is inserted, into the tube (53) of the perfusion circuit (Fig. 8; Fig. 21) an element (71) represented by a closed chamber with a cap (72) having two accesses with the functions of inlet (211) and outlet (212) of the bubble-trap type (Fig. 21); said chamber having a volume, a first part thereof is filled with warm fresh culture medium and a second part thereof is filled with air so as to trap in the latter second part of volume any air bubbles present in the perfusion fluid flowing through said accesses (211 and 212).

RP5. The process according to any one of claims RP1-4, wherein the scaffold, preferably a tubular scaffold, is selected among natural or synthetic origin polymer scaffolds, formed of a single polymer or copolymers, such as for example electrospun silk fibroin or copolymers of PGA/PLA (polyglycolic acid/polylactic acid) or PGA/PCL (polyglycolic acid/polyprolactone).

RP6. The process according to any one of RP1-5, wherein the endothelial cells are selected among the cells constituting an endothelium of a vascular tissue, such as for example HAOECs (human aortic endothelial cells), HCAECs (human coronary artery endothelial cells), HMEVECs (human dermal microvascular endothelial cells) or HUVECs (human umbilical vein endothelial cells).

RP7. The process according to any one of RP1-6, wherein the culture medium is Endothelial Growth Medium (EGM, Sigma Aldrich, 211-500), preferably heated to 37°C.

RP8. A scaffold having a lumen coated with a continuous functional endothelium obtained by means of the process according to any of RP1-7.

RP9. Use of the scaffold according to RP8, to perform *in vitro* preclinical tests and clinical trials of medical products for use in the cardiovascular and peripheral vascular area, such as for instance heart valves, stents, grafts and catheters.

The first step to perform and optimise is the cell seeding step, preferably endothelial cells, followed by the second critical step of connecting the perfusion circuit to the system comprising the bioreactor and the scaffold, so as to assure reliability, efficacy and reproducibility of the industrialisation process for the *in vitro* generation of a continuous functional endothelium.

The success of the industrialisation process according to this invention for the production of engineered vascular tissue/construct, preferably a scaffold having a lumen coated with a functional continuous endothelium having a confluent cell monolayer, consists principally in the success of the seeding and the connection of the perfusion circuit to the bioreactor-scaffold system, so that the air bubbles are reliably and reproducibly eliminated from the entire system. The seeding method depends on the cell source and the density of the cells, the chemical properties and porosity of the scaffold and the total removal of air bubbles from the lumen of the scaffold during injection of the cell suspension. On the other hand, the method for connecting the perfusion circuit to the bioreactor-scaffold system is based on the maintained sterility of the assembled system and the guarantee that no air bubbles are able to come into contact with the seeded scaffold. Note that the formation of air bubbles must be avoided inasmuch as the air bubbles can damage the cells, compromising their vitality and hence preventing the endothelisation of the scaffold. The description of the present invention shows that the choice of the method for connecting the perfusion circuit to the bioreactor-scaffold system depends on the previously optimised method for seeding the endothelial cells since said method of connection must be adapted to the experimental setup and the requirements of perfusion, and on the choice of the position chosen for this system in the incubator.

The process for seeding a scaffold, preferably a tubular scaffold, is one of the crucial factors in the generation *in vitro* of functional engineered vascular constructs with confluent endothelium, as shown in the description of the present invention. This process is responsible for the uniform and homogeneous distribution of the endothelial cells in the lumen, as well as for the adhesion of said cells to the surface. The description of the present invention shows that the choice of the most suitable seeding method, adapted to any bioreactor-scaffold system, defines the reproducibility of the process, and demonstrates its advantages in the reproducibility of the results.

Even in preclinical tests, but not only, there is a need for reproducible methods for the large scale production of functional vascular constructs.

It follows that the seeding method used in the present invention, well defined and traceable, assures a highly uniform distribution in the adhesion of the endothelial cells and a good reproducibility of the results, necessary for a laboratory the work of which is focused on the production *in vitro* of vascular constructs as models for industrial testing, not only preclinical.

After 24 hours of adhesion, the cells adhering to the lumen of the scaffold, principally tubular, must maintain their morphology and vitality to enable the formation of a homogeneous vascular endothelium. It follows that it is important to prevent any cell alteration during the connection process capable of modifying the adhesion of the cells, thus losing the growing vascular layer (as it is forming).

The known methods for connecting the bioreactor with the perfusion circuit compromise the endothelial cells and cause them to detach, even partially, from the luminal surface, so that the formation of a functional endothelial layer is slowed down or impeded. Furthermore, the known methods for connecting the bioreactor to the perfusion circuit do not assure the absence of any air bubbles, which may be formed by the compression or twisting (total or partial) of the connection tubes during perfusion. It is important to totally avoid contact between said air bubbles and the seeded scaffold by introducing an element, for example a bubble-trap, capable of eliminating the air bubbles before they reach the seeded scaffold. This disadvantage has been overcome successfully by the process according to the present invention, which obtains an internal surface of the scaffold coated with a uniform and functional layer of endothelial cells, in particular a confluent cell layer.

## Claims

1. A process for the production of an engineered vascular tissue or construct comprising a scaffold (21) having a lumen coated with a functional and continuous endothelium having a confluent cell monolayer, for testing medical or veterinary products, said process comprising the application of:
- a method for seeding an endothelial cell culture into the lumen of a scaffold (21) to yield a seeded scaffold (21); said seeded scaffold (21) being present inside a bioreactor (11), to yield a seeded bioreactor (11)-scaffold (21) system;
wherein said seeding method comprises the steps of:
- releasing said endothelial cell culture in the form of a cell suspension comprising a fresh culture medium and endothelial cells inside a container (91) mounted on a T connector (T2) located upstream of the bioreactor (11) by a rotary connector (CR1); followed by
- releasing said endothelial cell culture into the lumen of the scaffold (21) inside the bioreactor chamber (11) with a continuous flow such that the flow velocity permits said cell suspension to descend into the T connector (T2) without generating air bubbles and push the air bubbles inside the lumen of the scaffold (21) towards an opening of a T connector (T3) located downstream of the bioreactor (11) enabling them to exit;
and successively,
- a perfusion method with a fresh culture medium having a temperature between 30°C and 45°C, preferably 37°C, of the endothelial cells present in the lumen of said seeded scaffold (21); said perfusion method being realised by the connection of a perfusion circuit (51-56) or (51-57 and BT) to said seeded bioreactor(11)-scaffold (21) system;
wherein said perfusion method comprises a step of:
- partly filling an element for the removal of the air bubbles (71) or (BT) present in the perfusion circuit with said fresh culture medium, wherein said element for the removal of the bubbles (71) or (BT) comprises a chamber, a cap closing said chamber, an inlet access (211) and an outlet access (212), wherein said element for the removal of the air bubbles (71 or BT) has a volume and wherein a first part of said volume is filled with said fresh culture medium and wherein a second part of said volume is filled with air, said second part of said volume having the function of trapping the air bubbles present in said fresh culture
medium which flows through said inlet access (211) and said outlet access (212).

2. The process according to claim 1, wherein said seeding method of said endothelial cell culture in the lumen of said scaffold (21) comprises:
- mounting the scaffold (21), preferably a tubular scaffold of electrospun silk fibroin, on the mountings of a scaffold support (13, 13a, 13b) and seating said scaffold support (13, 13a, 13b) with the scaffold (21) inside the bioreactor chamber (11), to yield a bioreactor(11)-scaffold (21) system;
followed by
- injecting the fresh culture medium into the lumen of said scaffold (21) fastened on said scaffold support (13) inside the bioreactor chamber (11); followed by
- adding said fresh culture medium inside the bioreactor chamber (11) in which is present said scaffold support (13, 13a, 13b) with the scaffold (21) injected with said culture medium; followed by
- leaving, for an interval of time between 1 hour and 18 hours at a temperature between 20°C and 30°C, preferably 25°C, said culture medium inside the lumen of the scaffold (21) and inside the bioreactor chamber (11) in which is present said scaffold support (13) with the scaffold (21) injected with said culture medium; followed by
- clearing the interior of the lumen of the scaffold (21) and of the bioreactor chamber (11) of the culture medium; followed by
- releasing said endothelial cell culture inside said container (91) according to claim 1, preferably said container (91) being a syringe; followed by
- releasing said cell suspension into the lumen of the scaffold (21) according to claim 1; followed by
- adding said fresh culture medium inside the bioreactor chamber (11) in which is present said scaffold support (13) with the seeded scaffold (21) containing said celi suspension in its lumen; and followed by
- incubating, preferably for 24 hours at 37°C in the presence of 5% CO₂, the scaffold (21) seated inside the bioreactor chamber (11).

3. The process according to claims 1 or 2, wherein said perfusion method of the endothelial cells in the lumen of said seeded scaffold (21) comprises:
- providing said closed perfusion circuit comprising tubes (51), (52), (53), (54) and optionally (55);
- occluding the tube (54) or (55) of the perfusion circuit with a closing element (171) located proximally to a connector (C), preferably said closing element is a clamp or analogous device; followed by
- unscrewing the connector (C) located between the tube (53) or (54) and the tube (54) or (55) respectively of the perfusion circuit;
- screwing the tube (53) or (54) of the perfusion circuit to an open lateral end of the T connector (T2) upstream of the bioreactor (11) at a lateral access thereof; followed by
- opening the T connector (T3) downstream of the bioreactor (11) and unscrewing a cap of a lateral opening of the T connector (T3); followed by
- connecting the tube (54) or (55) of the perfusion circuit to the lateral opening of the T connector (T3) located downstream of the bioreactor (11) and removing the closing element (171);
- inserting, between the tube (53) and the pump loop tubing (52) of the perfusion circuit, the element for the removal of the air bubbles (71).

4. The process according to any of the preceding claims, wherein the element for the removal of the air bubbles (71) or (BT) is a bubble-trap or analogous device.

5. The process according to any one of the preceding claims, wherein the scaffold (21), preferably a tubular scaffold, is selected among synthetic or natural origin polymer scaffolds, wherein said polymer scaffolds are formed of a single polymer or copolymers, preferably electrospun silk fibroin or copolymers of polyglycolic acid I polylactic acid (PGAIPLA) or polyglycolic I polycaprolactone diacid copolymers (PGA/PCL).

6. The process according to any one of the preceding claims, wherein the endothelial cells are selected among the cells constituting an endothelium of a vascular tissue, preferably HAOECs (human aortic endothelial cells), HCAECs (human coronary artery endothelial cells), HMEVECs (human dermal microvascular endothelial cells) or HUVECs (human umbilical vein endothelial cells).

7. The process according to any one of the preceding claims, wherein the culture medium is Endothelial Growth Medium comprising foetal bovine serum (2%), adenine (0.2 µg/ml), ammonium metavanadate (0.0006 µg/ml), amphotericin B (0.3 µg/ml), calcium chloride 2H₂0 (300 µg/ml), choline hydrochloride (20 µg/ml), copper sulphate 5H₂0 (0.002 µg/ml), trioptic acid DL-6,8(0.003 µg/ml), folinic acid (calcium) (0.6 µg/ml), heparin (4 µg/ml), hydrocortisone (2 µg/ml), L-aspartic acid (15 µg/ml), L-cysteine (30 µg/ml), L- tyrosine (20 µg/ml), manganous sulphate monohydrate (0.0002 µg/ml), ammonium molybdate 4H₂0 (0.004 µg/ml), nicotinamide (8 µg/ml), nickel chloride 6H₂0 (0.0001 µg/ml), penicillin (60 µg/ml), phenol red sodium salt (15 µg/ml), potassium chloride (300 µg/ml), putrescine dihydrochloride (0.0002 µg/ml), pyridoxine hydrochloride (3 µg/ml), sodium metasilicate 9H₂O (3 µg/ml), sodium sulphate 7H₂O (200 µg/ml), sodium selenite (0.01 µg/ml), streptomycin sulphate (100 µg/ml), thiamine hydrochloride (4 µg/ml) and zinc sulphate 7H₂O (0.0003 µg/ml), preferably heated to 37°C.

## Patentansprüche

1. Verfahren zur Herstellung eines künstlichen Gefäßgewebes oder -konstrukts, umfassend ein Gerüst (21) aufweisend ein Lumen, das mit einem funktionellen und kontinuierlichen Endothel aufweisend eine konfluente Zellmonolage beschichtet ist, zum Testen von medizinischen oder veterinärmedizinischen Produkten, wobei das Verfahren die Anwendung umfasst von:
- einer Methode zum Aussäen einer Endothelzellkultur in das Lumen eines Gerüsts (21), um ein ausgesätes Gerüst (21) zu erhalten; wobei das ausgesäte Gerüst (21) innerhalb eines Bioreaktors (11) vorhanden ist, um ein ausgesätes Bioreaktor (11)-Gerüst (21) System zu erhalten;
wobei die Aussaatmethode die folgenden Schritte umfasst:
- Freisetzen der Endothelzellkultur in Form einer Zellsuspension, die ein frisches Kulturmedium und Endothelzellen umfasst, innerhalb eines Behälters (91), der an einem T-Verbinder (T2) angebracht ist, der sich stromaufwärts des Bioreaktors (11) durch einen Drehverbinder (CR1) befindet; gefolgt von
- Freisetzen der Endothelzellkultur in das Lumen des Gerüsts (21) innerhalb der Bioreaktorkammer (11) mit einer kontinuierlichen Strömung, so dass die Strömungsgeschwindigkeit es der Zellsuspension ermöglicht, in den T-Verbinder (T2) abzusteigen, ohne Luftblasen zu erzeugen und die Luftblasen innerhalb des Lumens des Gerüsts (21) in Richtung einer Öffnung eines T-Verbinders (T3) zu drücken, der sich stromabwärts des Bioreaktors (11) befindet, und ihnen ermöglicht, auszutreten;
und danach
- eine Perfusionsmethode mit einem frischen Kulturmedium aufweisend eine Temperatur zwischen 30 °C und 45 °C, vorzugsweise 37 °C, der Endothelzellen, die im Lumen des ausgesäten Gerüsts (21) vorhanden sind; wobei die Perfusionsmethode durch die Verbindung eines Perfusionskreislaufs (51-56) oder (51-57 und BT) mit dem ausgesäten Bioreaktor (11)-Gerüst (21) System realisiert wird;
wobei die Perfusionsmethode einen Schritt umfasst, zum:
- teilweisen Befüllen eines Elements zum Entfernen der in dem Perfusionskreislauf vorhandenen Luftblasen (71) oder (BT) mit dem frischen Kulturmedium, wobei das Element zum Entfernen der Luftblasen (71) oder (BT) eine Kammer, eine die Kammer verschließende Kappe, einen Einlasszugang (211) und einen Auslasszugang (212) umfasst, wobei das Element zum Entfernen der Luftblasen (71 oder BT) ein Volumen aufweist und wobei ein erster Teil des Volumens mit dem frischen Kulturmedium gefüllt ist und wobei ein zweiter Teil des Volumens mit Luft gefüllt ist, wobei der zweite Teil des Volumens die Funktion hat, die Luftblasen, die im frischen Kulturmedium vorhanden sind, das durch den Einlasszugang (211) und den Auslasszugang (212) strömt, einzufangen.

2. Verfahren nach Anspruch 1, wobei die Aussaatmethode der Endothelzellkultur im Lumen des Gerüsts (21) umfasst:
- Montieren des Gerüsts (21), vorzugsweise eines rohrförmigen Gerüsts aus elektrogesponnenem Seidenfibroin, auf den Halterungen eines Gerüstträgers (13, 13a, 13b) und Aufsetzen des Gerüstträgers (13, 13a, 13b) mit dem Gerüst (21) innerhalb der Bioreaktorkammer (11), um ein Bioreaktor (11)-Gerüst (21) System zu erhalten;
gefolgt von
- Injizieren des frischen Kulturmediums in das Lumen des auf dem Gerüstträger (13) befestigten Gerüsts (21) innerhalb der Bioreaktorkammer (11); gefolgt von
- Zugeben des frischen Kulturmediums innerhalb der Bioreaktorkammer (11), in der sich der Gerüstträger (13, 13a, 13b) mit dem mit dem Kulturmedium injizierten Gerüst (21) befindet; gefolgt von
- Belassen des Kulturmediums für einen Zeitraum zwischen 1 Stunde und 18 Stunden bei einer Temperatur zwischen 20 °C und 30 °C, vorzugsweise 25 °C, innerhalb des Lumens des Gerüsts (21) und innerhalb der Bioreaktorkammer (11), in der sich der Gerüstträger (13) befindet, wobei das Gerüst (21) mit dem Kulturmedium injiziert wird; gefolgt von
- Reinigen des Inneren des Lumens des Gerüsts (21) und der Bioreaktorkammer (11) des Kulturmediums; gefolgt von
- Freisetzen der Endothelzellkultur innerhalb des Behälters (91) nach Anspruch 1, wobei der Behälter (91) vorzugsweise eine Spritze ist; gefolgt von
- Freisetzen der Zellsuspension in das Lumen des Gerüsts (21) nach Anspruch 1; gefolgt von
- Zugeben des frischen Kulturmediums in die Bioreaktorkammer (11), in der sich der Gerüstträger (13) befindet, mit dem ausgesäten Gerüst (21), das die Zellsuspension in seinem Lumen enthält; und gefolgt von
- Inkubieren, vorzugsweise für 24 Stunden bei 37 °C in Gegenwart von 5% CO₂, des Gerüsts (21), das innerhalb der Bioreaktorkammer (11) sitzt.

3. Verfahren nach Anspruch 1 oder 2, wobei die Perfusionsmethode der Endothelzellen im Lumen des ausgesäten Gerüsts (21) umfasst:
- Bereitstellen des geschlossenen Perfusionskreislaufs, der Schläuche (51), (52), (53), (54) und optional (55) umfasst;
- Verschließen des Schlauches (54) oder (55) des Perfusionskreislaufes mit einem Verschlusselement (171), das sich proximal zu einem Verbinder (C) befindet, wobei das Verschlusselement vorzugsweise eine Klemme oder eine ähnliche Vorrichtung ist; gefolgt von
- Abschrauben des Verbinders (C), der sich zwischen dem Schlauch (53) oder (54) und dem Schlauch (54) bzw. (55) des Perfusionskreislaufs befindet;
- Verschrauben des Schlauches (53) oder (54) des Perfusionskreislaufs mit einem offenen seitlichen Ende des T-Verbinders (T2) stromaufwärts des Bioreaktors (11) an einem seitlichen Zugang desselben; gefolgt von
- Öffnen des T-Verbinders (T3) stromabwärts des Bioreaktors (11) und Abschrauben einer Kappe einer seitlichen Öffnung des T-Verbinders (T3); gefolgt von
- Verbinden des Schlauchs (54) oder (55) des Perfusionskreislaufs mit der seitlichen Öffnung des T-Verbinders (T3), der sich stromabwärts des Bioreaktors (11) befindet, und Entfernen des Verschlusselements (171);
- Einführen des Elements zum Entfernen der Luftblasen (71) zwischen dem Schlauch (53) und der Pumpenschleifenverschlauchung (52) des Perfusionskreislaufs.

4. Verfahren nach einem der vorhergehenden Ansprüche, wobei das Element zum Entfernen der Luftblasen (71) oder (BT) eine Blasenfalle oder eine ähnliche Vorrichtung ist.

5. Verfahren nach einem der vorhergehenden Ansprüche, wobei das Gerüst (21), vorzugsweise ein rohrförmiges Gerüst, aus Polymergerüsten synthetischen oder natürlichen Ursprungs ausgewählt ist, wobei die Polymergerüste aus einem einzelnen Polymer oder Copolymeren, vorzugsweise elektrogesponnenem Seidenfibroin oder Copolymeren von Polyglykolsäure / Polymilchsäure (PGA/PLA) oder Polyglykol/Polycaprolactondisäure-Copolymeren (PGA/PCL), gebildet sind.

6. Verfahren nach einem der vorhergehenden Ansprüche, wobei die Endothelzellen aus den Zellen ausgewählt werden, die ein Endothel eines Gefäßgewebes bilden, vorzugsweise HAOECs (humane Aortenendothelzellen), HCAECs (humane Koronararterien-Endothelzellen), HMEVECs (humane dermale mikrovaskuläre Endothelzellen) oder HUVECs (humane Nabelvenen-Endothelzellen).

7. Verfahren nach einem der vorhergehenden Ansprüche, wobei das Kulturmedium endotheliales Wachstumsmedium ist, umfassend fötales Rinderserum (2%), Adenin (0,2 µg/ml), Ammoniummetavanadat (0,0006 µg/ml), Amphotericin B (0,3 µg/ml), Calciumchlorid 2H₂O (300 µg/ml), Cholinhydrochlorid (20 µg/ml), Kupfersulfat 5H₂O (0,002 µg/ml), Trioptinsäure DL-6,8 (0,003 µg/ml), Folinsäure (Calcium) (0,6 µg/ml), Heparin (4 µg/ml), Hydrocortison (2 µg/ml), L-Asparaginsäure (15 µg/ml), L-Cystein (30 µg/ml), L-Tyrosin (20 µg/ml), Mangansulfat-Monohydrat (0,0002 µg/ml), Ammoniummolybdat 4H₂O (0,004 µg/ml), Nicotinamid (8 µg/ml), Nickelchlorid 6H₂O (0,0001 µg/ml), Penicillin (60 µg/ml), Phenolrot-Natriumsalz (15 µg/ml), Kaliumchlorid (300 µg/ml), Putrescindihydrochlorid (0,0002 µg/ml), Pyridoxinhydrochlorid (3 µg/ml), Natriummetasilikat 9H₂O (3 µg/ml), Natriumsulfat 7H₂O (200 µg/ml)/ml, Natriumselenit (0,01 µg/ml), Streptomycinsulfat (100 µg/ml), Thiaminhydrochlorid (4 µg/ml) und Zinksulfat 7H₂O (0,0003 µg/ml), vorzugsweise auf 37 °C erwärmt.

## Revendications

1. Procédé de production d'un tissu ou d'une construction vasculaire reconstruit(e), comprenant un échafaudage (21) comportant une lumière recouverte d'un endothélium fonctionnel et continu comportant une monocouche cellulaire confluente, pour tester des produits médicaux ou vétérinaires, ledit procédé comprenant l'application :
- d'une méthode d'ensemencement d'une culture de cellules endothéliales dans la lumière d'un échafaudage (21) pour obtenir un échafaudage ensemencé (21) ; ledit échafaudage ensemencé (21) étant présent à l'intérieur d'un bioréacteur (11), pour produire un système bioréacteur (11)-échafaudage (21) ensemencé ;
dans lequel ladite méthode d'ensemencement comprend les étapes de :
- libérer ladite culture de cellules endothéliales sous la forme d'une suspension cellulaire comprenant un milieu de culture frais et des cellules endothéliales à l'intérieur d'un récipient (91) monté sur un connecteur en « T » (T2) situé en amont du bioréacteur (11) par un connecteur rotatif (CR1) ; puis
- libérer ladite culture de cellules endothéliales dans la lumière de l'échafaudage (21) à l'intérieur de la chambre du bioréacteur (11) avec un flux continu tel que la vitesse du flux permet à ladite suspension cellulaire de descendre dans le connecteur en « T » (T2) sans générer de bulles d'air et de pousser les bulles d'air à l'intérieur de la lumière de l'échafaudage (21) vers une ouverture d'un connecteur en « T » (T3) situé en aval du bioréacteur (11) pour leur permettre de sortir ; et successivement
- une méthode de perfusion avec un milieu de culture frais ayant une température comprise entre 30 et 45 °C, de préférence 37 °C, des cellules endothéliales présentes dans la lumière dudit échafaudage ensemencé (21) ; ladite méthode de perfusion étant réalisée par le raccordement d'un circuit de perfusion (51-56) ou (51-57 et BT) audit système bioréacteur ensemencé(11)-échafaudage (21) ;
dans lequel ladite méthode de perfusion comprend une étape de :
- remplir partiellement un élément pour l'élimination des bulles d'air (71) ou (BT) présentes dans le circuit de perfusion avec ledit milieu de culture frais, dans lequel ledit élément pour l'élimination des bulles (71) ou (BT) comprend une chambre, un bouchon fermant ladite chambre, un accès d'entrée (211) et un accès de sortie (212), dans lequel ledit élément pour l'élimination des bulles d'air (71 ou BT) a un volume et dans lequel une première partie dudit volume est remplie avec ledit milieu de culture frais et dans lequel une deuxième partie dudit volume est remplie d'air, ladite deuxième partie dudit volume ayant pour fonction de piéger les bulles d'air présentes dans ledit milieu de culture frais qui s'écoule à travers ledit accès d'entrée (211) et ledit accès de sortie (212).

2. Procédé selon la revendication 1, dans lequel ladite méthode d'ensemencement de ladite culture de cellules endothéliales dans la lumière dudit échafaudage (21) comprend :
- monter l'échafaudage (21), de préférence un échafaudage tubulaire de fibroïne de soie électrofilée, sur les montages d'un support d'échafaudage (13, 13a, 13b) et placer ledit support d'échafaudage (13, 13a, 13b) avec l'échafaudage (21) à l'intérieur de la chambre du bioréacteur (11), pour produire un système bioréacteur (11)-échafaudage (21) ;
puis
- injecter le milieu de culture frais dans la lumière dudit échafaudage (21) fixé sur ledit support d'échafaudage (13) à l'intérieur de la chambre du bioréacteur (11) ; puis
- ajouter ledit milieu de culture frais à l'intérieur de la chambre du bioréacteur (11) dans laquelle se trouve ledit support d'échafaudage (13, 13a, 13b) avec l'échafaudage (21) injecté avec ledit milieu de culture ; puis
- laisser, pendant un intervalle de temps compris entre 1 et 18 heures à une température comprise entre 20 et 30 °C, de préférence 25 °C, ledit milieu de culture à l'intérieur de la lumière de l'échafaudage (21) et à l'intérieur de la chambre du bioréacteur (11) dans laquelle se trouve ledit support d'échafaudage (13) avec l'échafaudage (21) injecté avec ledit milieu de culture ; puis
- nettoyer l'intérieur de la lumière de l'échafaudage (21) et de la chambre du bioréacteur (11) du milieu de culture ; puis
- libérer ladite culture de cellules endothéliales à l'intérieur dudit récipient (91) selon la revendication 1, de préférence ledit récipient (91) étant une seringue ; puis
- libérer ladite suspension cellulaire dans la lumière de l'échafaudage (21) selon la revendication 1 ; puis
- ajouter ledit milieu de culture frais à l'intérieur de la chambre du bioréacteur (11) dans laquelle se trouve ledit support d'échafaudage (13) avec l'échafaudage ensemencé (21) contenant ladite suspension de cellules dans sa lumière ; et puis
- incuber, de préférence pendant 24 heures à 37 °C en présence de 5 % de CO₂, de l'échafaudage (21) placé à l'intérieur de la chambre du bioréacteur (11).

3. Procédé selon les revendications 1 ou 2, dans lequel ladite méthode de perfusion des cellules endothéliales dans la lumière dudit échafaudage ensemencé (21) comprend :
- fournir ledit circuit de perfusion fermé comprenant des tubes (51), (52), (53), (54) et éventuellement (55) ;
- obstruer le tube (54) ou (55) du circuit de perfusion à l'aide d'un élément de fermeture (171) situé à proximité d'un connecteur (C), de préférence ledit élément de fermeture est une pince ou un dispositif analogue ; puis
- dévisser le connecteur (C) situé entre le tube (53) ou (54) et le tube (54) ou (55) respectivement du circuit de perfusion ;
- visser le tube (53) ou (54) du circuit de perfusion à une extrémité latérale ouverte du connecteur en « T » (T2) en amont du bioréacteur (11) en correspondance d'un accès latéral de celui-ci ; puis
- ouvrir le connecteur en « T » (T3) en aval du bioréacteur (11) et dévisser un capuchon d'une ouverture latérale du connecteur en « T » (T3) ; puis
- raccorder le tube (54) ou (55) du circuit de perfusion à l'ouverture latérale du connecteur en « T » (T3) situé en aval du bioréacteur (11) et retirer l'élément de fermeture (171) ;
- insérer, entre le tube (53) et la tubulure de la boucle de pompe (52) du circuit de perfusion, l'élément pour l'élimination des bulles d'air (71).

4. Procédé selon l'une quelconque des revendications précédentes, dans lequel l'élément pour l'élimination des bulles d'air (71) ou (BT) est un piège à bulles ou un dispositif analogue.

5. Procédé selon l'une quelconque des revendications précédentes, dans lequel l'échafaudage (21), de préférence un échafaudage tubulaire, est choisi parmi les échafaudages polymères synthétiques ou d'origine naturelle, lesdits échafaudages polymères étant formés d'un seul polymère ou de copolymères, de préférence de la fibroïne de soie électrofilée ou des copolymères d'acide polyglycolique / acide polylactique (PGA/PLA) ou des copolymères diacides polyglycoliques / polycaprolactone (PGA/PCL).

6. Procédé selon l'une quelconque des revendications précédentes, dans lequel les cellules endothéliales sont choisies parmi les cellules constituant un endothélium d'un tissu vasculaire, de préférence les HAOEC (cellules endothéliales de l'aorte humaine), les HCAEC (cellules endothéliales de l'artère coronaire humaine), les HMEVEC (cellules endothéliales microvasculaires dermiques humaines) ou les HUVEC (cellules endothéliales de la veine ombilicale humaine).

7. Procédé selon l'une quelconque des revendications précédentes, dans lequel le milieu de culture est un milieu de croissance endothéliale comprenant du sérum bovin fætal (2 %), de l'adénine (0,2 µg/ml), du métavanadate d'ammonium (0,0006 µg/ml), de l'amphotéricine B (0,3 µg/ml), du chlorure de calcium 2H₂O (300 µg/ml), du chlorhydrate de choline (20 µg/ml), du sulfate de cuivre 5H₂O (0,002 µg/ml), de l'acide trioptique DL-6,8(0,003 µg/ml), de l'acide folinique (calcium) (0,6 µg/ml), de l'héparine (4 µg/ml), de l'hydrocortisone (2 µg/ml), de l'acide L-aspartique (15 µg/ml), L-cystéine (30 µg/ml), L-tyrosine (20 µg/ml), du sulfate de manganèse monohydraté (0,0002 µg/ml), du molybdate d'ammonium 4H₂O (0,004 µg/ml), du nicotinamide (8 µg/ml), du chlorure de nickel 6H₂O (0,0001 µg/ml), de la pénicilline (60 µg/ml), du sel de sodium de rouge de phénol (15 µg/ml), du chlorure de potassium (300 µg/ml), du dihydrochlorure de putrescine (0,0002 µg/ml), du chlorhydrate de pyridoxine (3 µg/ml), du métasilicate de sodium 9H₂O (3 µg/ml), du sulfate de sodium 7H₂O (200 µg/ml), de la sélénite de sodium (0,01 µg/ml), du sulfate de streptomycine (100 µg/ml), du chlorhydrate de thiamine (4 µg/ml) et du sulfate de zinc 7H₂O (0,0003 µg/ml), de préférence chauffés à 37 °C.
